**Europäisches Patentamt**

**European Patent Office**

**Office européen des brevets**

(19)

(11) Veröffentlichungsnummer: **0 009 090**
**B1**

(12) # EUROPÄISCHE PATENTSCHRIFT

(45) Veröffentlichungstag der Patentschrift:
08.04.81

(51) Int. Cl.³: **C 07 C 175/00**

(21) Anmeldenummer: **79102526.5**

(22) Anmeldetag: **18.07.79**

(54) **Verfahren zur Reinigung von Vitamin-A.**

(30) Priorität: **26.07.78 DE 2832697**

(43) Veröffentlichungstag der Anmeldung:
**02.04.80 Patentblatt 80/7**

(45) Bekanntmachung des Hinweises auf die Patenterteilung:
**08.04.81 Patentblatt 81/14**

(84) Benannte Vertragsstaaten:
**CH DE FR GB**

(56) Entgegenhaltungen:
**US-A-3 290 341**

(73) Patentinhaber: **BASF Aktiengesellschaft,
Carl-Bosch-Strasse 38, D-6700 Ludwigshafen (DE)**

(72) Erfinder: **Schulz, Bernhard, Dr. Chem., Kurpfalzring 28,
D-6830 Schwetzingen (DE)**
Erfinder: **Grassner, Hans, Dr. Chem., Wielandtstrasse 4a,
D-6900 Heidelberg (DE)**
Erfinder: **Grafen, Paul, Dr. Chem., Suedtiroler Ring 18-20,
D-6719 Weisenheim (DE)**

Verfahren zur Reinigung von Vitamin-A

Die Erfindung betrifft ein Verfahren zur Extraktion von seifenbildenden freien Fettsäuren aus diese Fettsäuren in freier und veresterter Form enthaltenden Estern von Vitamin-A-Alkohol durch Behandlung mit einer Mischung aus wäßrigem Alkali und einem niedermolekularen Alkohol mit 1 bis 3 Kohlenstoffatomen.

Fettsäureester enthalten häufig freie Fettsäuren. Um reine und haltbare Produkte zu erhalten, müssen die freien Fettsäuren entfernt werden. Eine Abtrennung der Fettsäuren kann durch Destillation oder durch fraktionierte Kristallisation in geeigneten Lösungsmitteln erfolgen. Diese Verfahren sind jedoch aufwendig und nicht allgemein anwendbar. Bei thermisch instabilen Produkten ist ferner eine Destillation nicht ohne weiteres möglich. Eine quantitative Abtrennung der Fettsäuren durch Kristallisation wird nur selten gelingen. Auch eine Extraktion mit wäßrigen Basen ist im allgemeinen nicht durchführbar, da die sich bildenden Alkalisalze Seifen darstellen, die eine Phasentrennung durch Emulsionsbildung verhindern. Man erhält stabile Emulsionen, die sich nicht mehr trennen lassen.

Es wurde nun gefunden, daß man freie Fettsäuren mit 8 bis 20 Kohlenstoffatomen von Estern dieser Fettsäuren mit Vitamin-A-Alkohol in einfacher Weise abtrennen kann, wenn man die freien Fettsäuren mit einer Mischung von wäßrigem Alkali und einem oder mehreren der Alkohole Methanol, Äthanol, Isopropanol oder 1,2-Propandiol extrahiert.

Die Möglichkeit dieser Reinigung war überraschend, da Vitamin-A-Ester, insbesondere in Gegenwart von Alkoholen, alkaliempfindlich sind und eine zumindest teilweise Zersetzung oder Hydrolyse zu Vitamin-A-Alkohol zu befürchten gewesen wäre.

Die zu extrahierenden Fettsäuren haben vorzugsweise 8 bis 18 Kohlenstoffatome. Im einzelnen kommen Capryl-, Caprin-, Laurin-, Myristin- und Linolsäure und insbesondere Palmitinsäure, Stearinsäure und Ölsäure in Betracht.

Die Fettsäuren sind in den Vitamin-A-Estern in der Regel in Mengen von 0,5 bis 30 Gew.-% enthalten. Aber auch ein höherer Gehalt ist möglich. Als niedermolekulare Alkohole mit 1 bis 3 Kohlenstoffatomen sind im einzelnen Methanol, Äthanol, Isopropanol und Propylenglykol-1,2 zu nennen.

Als Extraktionsmittel kommen Mischungen von wäßrigem Alkali mit den genannten Alkoholen in einem Volumenverhältnis von 70 : 30 bis 30 : 70 in Betracht. Zweckmäßig verwendet man Mischungen, die aus etwa gleichen Raumteilen des wäßrigen Alkalis und der $C_1$- bis $C_3$-Alkohole bestehen.

Als Alkali kommen z. B. die Carbonate und vorzugsweise die Hydroxide von Natrium und vor allem Kalium in Betracht.

Das aus Alkalilauge und Alkoholen bestehende Extraktionsmittel wendet man zweckmäßig in einer solchen Menge an, daß mindestens ein 5%iger Überschuß über die Menge Alkali vorliegt, die der stöchiometrischen Menge der zu extrahierenden Säure entspricht. Vorteilhaft wird ein 5- bis 100%iger Überschuß angewandt. Größere Mengen Alkali sind möglich, bringen jedoch keinen weiteren Vorteil.

Man führt die Extraktion bei Temperaturen zwischen 0°C und dem Siedepunkt der $C_1$- bis $C_3$-Alkohole aus, vorzugsweise bei 15 bis 50°C und insbesondere bei 30 bis 40°C.

Im allgemeinen löst man die zu reinigenden Vitamin-A-Ester zunächst in einem Kohlenwasserstoff oder Chlorkohlenwasserstoff mit einem Siedepunkt im Bereich von z. B. 50 bis 150°C und extrahiert diese Lösung. Nach Abtrennung des Alkali-/Alkanolgemisches erhält man den gereinigten Vitamin-A-Ester durch Verdampfen des Kohlenwasserstoffs bzw. Chlorkohlenwasserstoffs.

Die Extraktion kann in an sich bekannter Weise unter Verwendung üblicher Vorrichtungen ausgeführt werden. Im allgemeinen genügt das Ausschütteln oder bei größeren Ansätzen das Ausrühren.

Die Trennung der Phasen erfolgt im allgemeinen in 0,5 bis 10 Minuten. Die Trennungszeit ist abhängig von der Ansatzgröße und von der Temperatur. Bei höherer Temperatur trennen sich die Phasen schneller. Die Extraktion kann auch kontinuierlich als Gegenstromextraktion in Kolonnen mit und ohne Energiezufuhr, in Kolonnen mit rotierenden Einbauten und in Mischer-Abscheidern oder in anderen beliebigen Extraktoren durchgeführt werden. Es empfiehlt sich, mit Alkohol-Wasser-Mischungen oder mit reinem oder kohlensäurehaltigem Wasser nachzuwaschen, damit keine Alkalispuren in dem zu extrahierenden Produkt verbleiben.

Mit Hilfe des erfindungsgemäßen Verfahrens können die freien Fettsäuren quantitativ aus den entsprechenden Vitamin-A-Estern abgetrennt werden.

Auch kann man aus dem alkalischen Extrakt durch Ansäuern mit Mineralsäuren die freien Fettsäuren freisetzen und mit organischen Lösungsmitteln, wie Heptan, Toluol, Äther, diese Fettsäuren extrahieren oder die auskristallisierenden Fettsäuren abfiltrieren. So können die freien Fettsäuren leicht wiedergewonnen werden, falls dies erwünscht ist.

Ferner ergibt sich als weiterer Vorteil, daß Schwermetallspuren, die z. B. in Form der fettsauren Salze vorliegen können, als Hydroxide gefällt und mit der wäßrigen Phase entfernt werden.

Aus der US-Patentschrift 3 290 341 ist zwar die Entfernung von Verunreinigung aus Vitamin-A-Fettsäureestern mit Alkoholen bekannt. Doch geschieht dies dort nach Umesterung mit wasserfreiem Alkali als Katalysator und anschließender Neutralisation mit Säure. Deshalb ist dort sowohl die Aufgabenstellung als auch die Lösung anders, da keine freien Fettsäuren entfernt werden und keine Mischung vom wäßrigem Alkali mit Alkoholen zu Extraktion Verwendung findet.

In den folgenden Beispielen verhalten sich Gewichtsteile zu Raumteilen wie Kilogramm zu Liter.

### Beispiel 1

100 Gewichtsteile Vitamin-A-Palmitat mit der Säurezahl 12 (entspricht einem Gehalt von etwa 5 Gewichtsteilen Palmitinsäure) werden in 300 Raumteilen Hexan gelöst. Diese Lösung wird bei 40°C zweimal mit einer Mischung aus 200 Raumteilen 1 n-Kalilauge und 200 Raumteilen Isopropanol extrahiert. Anschließend wird zweimal mit 200 Raumteilen Wasser nachgewaschen. Die Hexanlösung wird eingedampft. Das zurückbleibende Vitamin-A-Palmitat hat eine Säurezahl <0,1. Dünnschichtchromatographisch ist kein Retinol nachweisbar. Die Ausbeute ist quantitativ.

### Beispiel 2

100 Raumteile einer Lösung in Hexan von 25 Gewichtsteilen Vitamin-A-Palmitat mit einem Gehalt von ca. 2,5 Gewichtsteilen Palmitinsäure werden dreimal bei Raumtemperatur mit einer Mischung aus 50 Raumteilen 1 n-KOH und 50 Raumteilen Propylenglykol-1,2 extrahiert. Anschließend wird dreimal mit je 100 Raumteilen Wasser nachgewaschen. Die Hexanlösung wird eingedampft. Das zurückbleibende Vitamin-A-Palmitat hat eine Säurezahl 0,1 und ist frei von Retinol. Die Ausbeute ist quantitativ. Das eingesetzte Vitamin-A-Palmitat hatte eine Säurezahl von 20,2. Eine Temperaturerhöhung bei der Extraktion auf 35 bis 50°C beschleunigt die Trennung der Phasen.

### Beispiel 3

Man verfährt wie in Beispiel 2 angegeben, verwendet jedoch Methanol statt Propylenglykol-1,2. Das erhaltene Vitamin-A-Palmitat hat eine Säurezahl von 0,14 und enthält geringe Spuren von Retinol.

### Beispiel 4

Man verfährt wie in Beispiel 3, verwendet aber Äthanol statt Propylenglykol-1,2. Das erhaltene Vitamin-A-Palmitat hat eine Säurezahl von 0,17 und ist praktisch frei von Retinol.

## Patentansprüche

1. Verfahren zur Abtrennung von freien Fettsäuren mit 8 bis 20 Kohlenstoffatomen von Estern des Vitamin-A-Alkohols mit diesen Fettsäuren, dadurch gekennzeichnet, daß man die freien Fettsäuren mit einer Mischung von wäßrigem Alkali und einem oder mehreren der Alkohole Methanol, Äthanol, Isopropanol oder 1,2-Propylenglykol bei Temperaturen zwischen 0°C und der Siedetemperatur des verwendeten Alkohols extrahiert.

2. Verfahren gemäß Anspruch 1, dadurch gekennzeichnet, daß man ein Volumenverhältnis von wäßrigem Alkali zu den Alkoholen von 70 : 30 bis 30 : 70 einhält.

3. Verfahren gemäß Anspruch 1, dadurch gekennzeichnet, daß die wäßrige Lösung 0,5 bis 2 Äquivalente Base pro Liter enthält.

4. Verfahren gemäß Anspruch 1, dadurch gekennzeichnet, daß man die Ester in einem flüssigen Kohlenwasserstoff oder Chlorkohlenwasserstoff löst und die Fettsäuren aus der Lösung extrahiert.

5. Verfahren gemäß Anspruch 1, dadurch gekennzeichnet, daß man als Extraktionsmittel eine Mischung aus wäßriger Kalilauge und Isopropanol verwendet.

## Claims

1. A process for removing free fatty acids of 8 to 20 carbon atoms from esters of vitamin A alcohol with these fatty acids, charae erized in that the free fatty acis are extracted with a mixture of an aqueous alkali and one or more of the alcohols methanol, ethanol, isopropanol and 1,2-propylene glycol at a temperature of from 0°C to the boiling point of the alcohol used.

2. A process as claimed in claim 1, characterized in that the volumetric ratio of aqueous alkali to alcohols is from 70 : 30 to 30 : 70.

3. A process as claimed in claim 1, characterized in that the aqueous solution contains from 0,5 to 2 equivalents of base per liter.

4. A process as claimed in claim 1, characterized in that the esters are dissolved in a liquid hydrocarbon or chlorohydrocarbon and the fatty acids are extracted from the solution.

5. A process as claimed in claim 1, characterized in that a mixture of aqueous potassium hydroxide solution and isopropanol is used as the extractant.

## Revendications

1. Procédé pour séparer les acides gras libres contenant de 8 à 20 atomes de carbone des esters de la vitamine A (alcool) et de ces acides gras, caractérisé en ce que l'on extrait les acides gras libres par un mélange d'alcali aqueux et d'un ou plusieurs des alcools méthanol, éthanol, isopropanol ou 1,2-propylène-glycol à des températures comprises entre 0°C et la température d'ébullition de l'alcool utilisé.

2. Procédé selon la revendication 1 caractérisé en ce que l'on respecte des proportions en volume de 70 : 30 à 30 : 70 entre l'alcali aqueux et les alcools.

3. Procédé selon la revendication 1, caractérisé en ce que la solution aqueuse contient de 0,5 à 2 équivalents de base par litre.

4. Procédé selon la revendication 1, caractérisé en ce que l'on les esters dans un hydrocarbure ou hydrocarbure chloré liquide et on extrait les acides gras de la solution.

5. Procédé selon la revendication 1, caractérisé en ce que l'on utilise comme agent d'extraction un mélange d'une lessive aqueuse de potasse et d'isopropanol.

4